# EUROPÄISCHE PATENTSCHRIFT

(11) **EP 3 016 632 B1**
(45) Veröffentlichungstag und Bekanntmachung des Hinweises auf die Patenterteilung: **20.12.2017**
(21) Anmeldenummer: 14742459.2
(22) Anmeldetag: 23.06.2014
(51) Int. Cl.: A61K 8/36, A61Q 5/02, A61K 8/86, A61Q 19/10, A61K 8/04, B01F 17/00, A61K 8/44, A61K 8/46, A61Q 5/12, A61Q 19/00

(54) **REINIGUNGSZUSAMMENSETZUNG MIT HOHEM FETTSÄUREGEHALT**
CLEANSING COMPOSITIONS CONTAINING HIGH LEVEL OF FATTY ACIDS
COMPOSITIONS DE NETTOYAGE AVEC UNE TENEUR ELEVEE EN ACIDES GRAS

(30) Priorität: 02.07.2013 DE 102013212873
(43) Veröffentlichungstag der Anmeldung: 11.05.2016
(73) Patentinhaber: Henkel AG & Co. KGaA, 40589 Düsseldorf (DE)
(72) Erfinder: SCHELGES, Heike, 47877 Willich (DE); DOMFELD, Evelyn, 40593 Düsseldorf (DE)
(86) Internationale Anmeldenummer: PCT/DE2014/200276
(87) Internationale Veröffentlichungsnummer: WO 2015/000478

(56) Entgegenhaltungen:
- EP-A2- 2 314 270
- WO-A1-01/13877
- WO-A1-2013/012420
- WO-A2-02/05758
- WO-A2-2008/038147
- DE-A1- 10 156 674

## Beschreibung

Die vorliegende Erfindung betrifft eine kosmetische Reinigungszusammensetzung mit einem hohen Fettsäuregehalt, welche hervorragende Schaumeigenschaften sowie eine mousseartige Textur aufweist.

Weiterhin betrifft die vorliegende Erfindung die Verwendung der erfindungsgemäßen Reinigungszusammensetzung zur Reinigung und Pflege von Haut und Haaren.

Reinigungszusammensetzungen, beispielsweise kosmetische Reinigungszusammensetzungen für die Haut und die Haare, wie flüssige Seifen, Shampoos, Duschbäder, Schaumbäder, Dusch- und Waschgele, müssen neben einem guten Reinigungsvermögen auch pflegende Eigenschaften aufweisen, um eine zu starke Entfettung oder Hauttrockenheit bei häufiger Anwendung zu vermeiden. Weiterhin sollen die Reinigungszusammensetzungen optisch und sensorisch ansprechend sein und eine angenehme Handhabung aufweisen.

Besonders Reinigungszusammensetzungen, welche eine leichte Textur in Form einer Mousse aufweisen sind optisch und sensorisch ansprechend, zeichnen sich durch eine einfache Anwendung aus und weisen aufgrund der leichten Verteilbarkeit ein hohes Reinigungsvermögen auf. Darüber hinaus ziehen die Inhaltsstoffe der Mousse schnell ein, so dass sich die pflegenden Eigenschaften erhöhen. Zudem resultiert kein glitschiges oder klebriges Gefühl auf der Anwendungsoberfläche nach dem Abspülen der Mousse.

Um eine zu starke Entfettung oder Hauttrockenheit bei häufiger Anwendung zu vermeiden, werden Rückfettungsmittel wie geradkettige oder verzweigte Carbonsäuren in die Mousse eingearbeitet, um den Anforderungen des Verbrauchers hinsichtlich der gleichzeitigen Reinigung und Pflege entgegen zu kommen. Nicht- bzw. teilneutralisierte Carbonsäuren senken jedoch das Schaumvermögen und damit das Reinigungsvermögen der Mousse, was vom Verbraucher nicht akzeptiert wird.

Deshalb wurden im Stand der Technik Reinigungszusammensetzungen entwickelt, welche eine mousseartige Textur bei Verwendung von nicht- bzw. teilneutralisierten Fettsäuren als Rückfettungsmittel aufweisen. So beschreibt die internationale Patentanmeldung WO 2013/012420 A1 wässrige Reinigungszusammensetzungen mit mousseartiger Textur, welche neben Fettsäureseifen auch nichtneutralisierte Fettsäuren enthalten. Die mousseartige Textur wird durch Verwendung von zweiwertigen Metallkationen erreicht, welche ein Gas in den Reinigungszusammensetzungen einschließen. Jedoch geht die Verwendung von Fettsäureseifen in Kombination mit zweiwertigen Metallkationen mit der Bildung schwerlöslicher Kalkseifen einher, welche optisch wenig ansprechende Ablagerungen auf der Haut und den Haaren sowie anderen Oberflächen verursachen und die Reinigungsleistung der Mousse verringern.

Es besteht daher ein Bedarf nach kosmetischen Haut- und Haarreinigungszusammensetzungen, welche eine mousseartige Textur aufweisen und gleichzeitig hervorragend reinigen. Darüber hinaus sollen sie hervorragende Schaumeigenschaften aufweisen, welche sich in der Schaummenge, einem stabilen Schaum sowie verbesserten sensorischen und optischen Eigenschaften des Schaums niederschlagen. Zudem sollen die kosmetischen Haut- und Haarreinigungszusammensetzungen einen hohen Anteil an rückfettenden nicht- bzw. teilneutralisierten Carbonsäuren aufweisen, um die pflegenden Eigenschaften zu erhöhen.

Der vorliegenden Erfindung lag die Aufgabe zugrunde, kosmetische Reinigungszusammensetzungen bereitzustellen, welche hervorragende Schaumeigenschaften aufweisen, die Haut und das Haar schonend und gründlich reinigen sowie aufgrund des hohen Anteils an rückfettenden und nicht- bzw. teilneutralisierten Carbonsäuren hervorragend pflegen.

Es wurde nun gefunden, dass der Einsatz von hochmolekularen nichtionischen Polymeren zu Reinigungszusammensetzungen führt, welche trotz des hohen Anteils an rückfettenden und nicht- bzw. teilneutralisierten Carbonsäuren hervorragende Schaum-, Reinigungs- und Pflegeeigenschaften aufweisen.

Gegenstand der vorliegenden Erfindung ist somit eine kosmetische Reinigungszusammensetzung, enthaltend
a) 0,1 bis 30 Gew.-%, bezogen auf das Gesamtgewicht der Reinigungszusammensetzung, Tensid, ausgewählt aus der Gruppe der anionischen, nichtionischen und/oder zwitterionischen Tenside sowie deren Mischungen,
b) mindestens eine nichtneutralisierte, unverzweigte, gesättigte und/oder ungesättigte Carbonsäure mit einer Kettenlänge von 14 bis 30 Kohlenstoffatomen in einer Menge von 11 bis 22 Gew.-% bezogen auf das Gesamtgewicht der Reinigungszusammensetzung und
c) mindestens ein nichtionisches Polymer der Formel (I) in einer Menge von 0,1 bis 1 Gew.-% bezogen auf das Gesamtgewicht der Reinigungszusammensetzung, 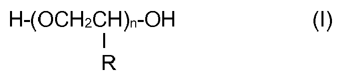
   wobei R für ein Wasserstoffatom oder eine Methylgruppe und n für einen ganzzahligen
   Wert von 1.360 bis 99.000 steht.

Die Reinigungszusammensetzungen der vorliegenden Erfindung liegen in Form einer Mousse mit optisch schöner Textur vor und weisen eine genügend hohe Stabilität auf, um als Mousse verpackt, gelagert und in den Handel gebracht zu werden. Die Mousse ist stabil und lässt sich leicht auf der Haut / den Haaren verteilen. Durch die geringe Porengröße des Schaums der Mousse fühlt sie sich cremig an, was den Pflegeeffekt während des Reinigungsvorgangs spürbar werden lässt. Des Weiteren lässt sich die Mousse nach der Reinigung schnell und gründlich wieder abspülen, ohne ein glitschiges oder klebriges Gefühl auf der Anwendungsoberfläche zu hinterlassen. Zudem weisen die erfindungsgemäßen Reinigungszusammensetzungen aufgrund des hohen Anteils an rückfettenden nicht- bzw. teilneutralisierten Carbonsäuren einen hohen pflegenden Charakter auf, so dass eine zu starke Entfettung oder Hauttrockenheit bei häufiger Anwendung vermieden wird.

Unter dem Begriff der Mousse, wie er erfindungsgemäß verwendet wird, sind Reinigungszusammensetzungen zu verstehen, welche nach dem Herstellprozess oder bei der Anwendung durch den Verbraucher eine schaumige, cremeartige Konsistenz aufweisen. Derartige Reinigungszusammensetzungen weisen eine beliebige Verteilung von mit Gas gefüllten kugel- oder polyederförmigen Zellen auf, welche durch flüssige, halbflüssige oder hochviskose Zellstege aus einer Mischung von Tensiden, Wasser und weiteren Hilfsstoffen begrenzt sind.

Weiterhin sind unter dem Begriff der Fettsäure, wie er im Rahmen der vorliegenden Erfindung verwendet wird, aliphatische Carbonsäuren zu verstehen, welche unverzweigte Kohlenstoffreste mit 4 bis 40 Kohlenstoffatomen aufweisen. Die im Rahmen der vorliegenden Erfindung eingesetzten Fettsäuren können sowohl natürlich vorkommende als auch synthetisch hergestellte Fettsäuren sein. Weiterhin können die Fettsäuren einfach oder mehrfach ungesättigt sein.

Darüber hinaus sind unter dem Begriff des Fettalkohols im Rahmen der vorliegenden Erfindung aliphatische, einwertige, primäre Alkohole zu verstehen, welche unverzweigte Kohlenwasserstoffreste mit 4 bis 40 Kohlenstoffatomen aufweisen. Die im Rahmen der Erfindung eingesetzten Fettalkohole können auch ein- oder mehrfach ungesättigt sein.

Um eine hervorragende Reinigungsleistung und besonders gute Schaumeigenschaften zu erzielen, wird in einer erfindungsgemäß bevorzugten Ausführungsform eine Mischung aus mindestens einem anionischen und mindestens einem nichtionischen und/oder zwitterionischen Tensid eingesetzt, wobei das Gewichtsverhältnis des(r) anionischen Tensids(e) zu dem(n) nichtionischen und/oder zwitterionischen Tensid(en) von 3 : 1 bis 1 : 2, bevorzugt 2 : 1 bis 1 : 1,5 und insbesondere 1,5 : 1 bis 1 : 1 beträgt.

In diesem Zusammenhang kann es zudem erfindungsgemäß vorgesehen sein, dass das anionische Tensid in der erfindungsgemäßen Reinigungszusammensetzung in einer Menge von 0,1 bis 50 Gew.-%, bevorzugt 0,2 bis 40 Gew.-%, mehr bevorzugt 0,3 bis 30 Gew.-%, noch mehr bevorzugt 0,4 bis 20 Gew.-% und insbesondere 0,5 bis 15 Gew.-%, bezogen auf das Gesamtgewicht der Reinigungszusammensetzung, enthalten ist.

Gemäß einer weiteren Ausführungsform der vorliegenden Erfindung kann das nichtionische und/oder zwitterionische Tensid in der erfindungsgemäßen Reinigungszusammensetzung in einer Menge von 0,1 bis 20 Gew.-%, bevorzugt 0,2 bis 18 Gew.-%, mehr bevorzugt 0,3 bis 15 Gew.-% und insbesondere 0,5 bis 10 Gew.-%, bezogen auf das Gesamtgewicht der Reinigungszusammensetzung, enthalten sein.

Als anionische Tenside können in den erfindungsgemäßen Reinigungszusammensetzungen alle für die Verwendung am menschlichen Körper geeigneten anionischen Tenside bzw. oberflächenaktiven Stoffe eingesetzt werden. Diese sind gekennzeichnet durch eine hydrophile anionische Gruppe, wie z. B. eine Carboxylat-, Sulfat-, Sulfonat- oder Phosphatgruppe und eine lipophile Alkylgruppe mit etwa 8 bis 30 Kohlenstoffatomen. Zusätzlich können die Tenside Glykol- oder Polyglykolethergruppen, Ester-, Ether- und Amidgruppen sowie Hydroxylgruppen aufweisen.

Erfindungsgemäß geeignete anionische Tenside sind, jeweils in Form der Natrium-, Kalium- und Ammonium- sowie der Mono-, Di- und Trialkanolammoniumsalze mit 2 bis 4 Kohlenstoffatomen in der Alkanolgruppe,
- lineare und verzweigte Fettsäuren mit 8 bis 30 Kohlenstoffatomen (Seifen),
- Ethercarbonsäuren der Formel (TI)

   R¹O-(CH₂CH₂O)_{X}-CH₂-COOH (TI)

   in der R¹ eine lineare Alkylgruppe mit 8 bis 30 Kohlenstoffatomen und x = 0 oder 1 bis 16 ist,
- Acylsarcoside mit 8 bis 24 Kohlenstoffatomen in der Acylgruppe,
- Acyltauride mit 8 bis 24 Kohlenstoffatomen in der Acylgruppe,
- Acylisethionate mit 8 bis 24 Kohlenstoffatomen in der Acylgruppe,
- Sulfobernsteinsäuremono- und -dialkylester mit 8 bis 24 Kohlenstoffatomen in der Alkylgruppe und Sulfobernsteinsäuremonoalkylpolyoxyethylester mit 8 bis Kohlenstoffatomen in der Alkylgruppe und 1 bis 6, bevorzugt 1 bis 4 Oxyethylgruppen,
- lineare Alkansulfonate mit 8 bis 24 Kohlenstoffatomen,
- lineare Alpha-Olefinsulfonate mit 8 bis 24 Kohlenstoffatomen,
- Alpha-Sulfonfettsäuremethylester von Fettsäuren mit 8 bis 30 Kohlenstoffatomen,
- Alkylsulfate und Alkylpolyglykolethersulfate der Formel (TII)

   R²-O(CH₂-CH₂O)ₓ-SO₃H (TII)

   in der R² eine bevorzugt lineare Alkylgruppe mit 8 bis 30 Kohlenstoffatomen und x = 0 oder 1 bis 14 ist,
- Gemische oberflächenaktiver Hydroxysulfonate,
- sulfatierte Hydroxyalkylpolyethylen- und/oder Hydroxyalkylenpropylenglykolether,
- Sulfonate ungesättigter Fettsäuren mit 8 bis 24 Kohlenstoffatomen und 1 bis 6 Doppelbindungen,
- Ester der Weinsäure und Zitronensäure mit Alkoholen, welche Anlagerungsprodukte von etwa 2 bis 15 Molekülen Ethylenoxid und/oder Propylenoxid an Fettalkohole mit 8 bis 22 Kohlenstoffatomen darstellen,
- Alkyl- und/oder Alkenyletherphosphate der Formel (TIII), 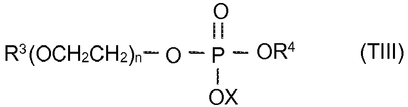 in der R³ bevorzugt ein aliphatischer Kohlenwasserstoffrest mit 8 bis 30 Kohlenstoffatomen, X Wasserstoff, ein Alkali- oder Erdalkalimetall oder NR⁵R⁶R⁷R⁸, mit R⁵ bis R⁸ unabhängig voneinander stehend für einen C₁- bis C₄-Kohlenwasserstoffrest, R⁴ Wasserstoff, ein Rest R³(CH₂CH₂O)ₙ oder X und n = 1 bis 10 ist,
- sulfatierte Fettsäurealkylenglykolester der Formel (TIV)

   R⁹CO-(Alk-O)ₙ-SO₃M (TIV)

   in der R⁹ für einen linearen oder verzweigten, aliphatischen, gesättigten und/oder ungesättigten Acylrest mit 6 bis 22 Kohlenstoffatomen, Alk für CH₂CH₂, CHCH₃CH₂ und/oder CH₂CHCH₃, n für Zahlen von 0,5 bis 5 und M für ein Kation steht, wie sie in der DE-OS 197 36 906.5 beschrieben sind,
- Monoglyceridsulfate und Monoglycerid(ether)sulfate der Formel (TV), 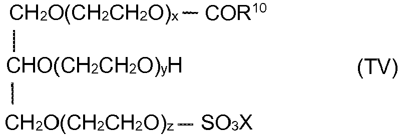
   in der R¹⁰ für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht.
   Typische Beispiele für im Sinne der Erfindung geeignete Monoglycerid(ether)sulfate in Form ihrer Natriumsalze sind die Umsetzungsprodukte von Laurinsäuremonoglycerid, Kokosfettsäuremonoglycerid, Palmitinsäuremonoglycerid, Stearinsäure-monoglycerid, Ölsäuremonoglycerid und Talgfettsäuremonoglycerid sowie deren Ethylen-oxidaddukte mit Schwefeltrioxid oder Chlorsulfonsäure.
   Vorzugsweise werden Monoglyceridsulfate der Formel (TV) eingesetzt, in der R¹⁰ für einen linearen Acylrest mit 8 bis 18 Kohlenstoffatomen steht.

Gemäß einer bevorzugten Ausführungsform der vorliegenden Erfindung ist das mindestens eine anionische Tensid ausgewählt aus der Gruppe der Alkylpolyglykolethersulfate, Alkylsulfate und/oder Sulfobernsteinsäuremonoalkylpolyoxyethylester mit 8 bis 18 Kohlenstoffatomen in der Alkylgruppe und 1 bis 10, bevorzugt 1 bis 4 Oxyethylgruppen in der Polyoxyethylgruppe. Die vorgenannten Tenside weisen besonders hautmilde Eigenschaften auf, so dass eine Reizung der Haut selbst bei häufiger Anwendung vermieden wird.

Erfindungsgemäß werden weiterhin nichtionische und/oder zwitterionische Tenside eingesetzt. Die nichtionischen Tenside weisen als hydrophile Gruppe z. B. eine Polyolgruppe, eine Polyalkylenglykolethergruppe oder eine Kombination aus Polyol- und Polyglykolethergruppe auf. Erfindungsgemäß geeignete nichtionische Tenside sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 Kohlenstoffatomen, an Fettsäuren mit 8 bis 30 Kohlenstoffatomen und an Alkylphenole mit 8 bis 15 Kohlenstoffatomen in der Alkylgruppe,
- mit einem Methyl- oder C₂- bis C₆-Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 Kohlenstoffatomen, an Fettsäuren mit 8 bis 30 Kohlenstoffatomen und an Alkylphenole mit 8 bis 15 Kohlenstoffatomen in der Alkylgruppe, wie beispielsweise die unter den Verkaufsbezeichnungen Dehydol® LS, Dehydol® LT (Cognis) erhältlichen Typen,
- C₁₂-C₃₀-Carbonsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen® HSP (Cognis) oder Sovermol-Typen (Cognis),
- alkoxilierte Triglyceride,
- alkoxilierte Fettsäurealkylester der Formel (TVI)

   R¹¹CO-(OCH₂CHR¹²)_{w}OR¹³ (TVI)

   in der R¹¹ für einen linearen oder verzweigten, gesättigten und/oder ungesättigten Acylrest mit 6 bis 22 Kohlenstoffatomen, R¹² für Wasserstoff oder Methyl, R¹³ für lineare oder verzweigte Alkylreste mit 1 bis 4 Kohlenstoffatomen und w für Zahlen von 1 bis 20 steht,
- Aminoxide,
- Hydroxymischether, wie sie beipielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Fettsäure-N-alkylglucamide.

Eine weitere Gruppe erfindungsgemäß geeigneter nichtionischer Tenside sind Alkylpolyglucoside. Sie entsprechen der Formel (TVII)

R¹⁴O-[G]ₚ (TVII)

in der R¹⁴ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Die Indexzahl p in der allgemeinen Formel (TVII) gibt den Oligomerisierungsgrad (DP), d. h. die Verteilung von Mono- und Oligoglucosiden, an und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkyloligoglucosid eine analytisch ermittelte rechnerische Größe, die meistens eine gebrochene Zahl darstellt. Erfindungsgemäß bevorzugt werden Alkyl- und/oder Alkenyloligoglucoside mit einem mittleren Oligomerisierungsgrad p von 1,1 bis 3,0 eingesetzt. Aus anwendungstechnischer Sicht sind solche Alkyl- und/oder Alkenyloligoglucoside bevorzugt, deren Oligomerisierungsgrad kleiner als 1,7 ist und insbesondere zwischen 1,2 und 1,7 liegt. Der Alkyl- bzw. Alkenylrest R¹⁴ kann sich von primären Alkoholen mit 4 bis 20, vorzugsweise 8 bis 16 Kohlenstoffatomen ableiten. Erfindungsgemäß ganz besonders bevorzugt sind Alkyloligoglucoside auf der Basis von gehärtetem C_{12/14}-Kokosalkohol mit einem DP von 1 bis 3, wie sie beispielsweise unter der INCI-Bezeichnung "Coco-Glucoside" im Handel erhältlich sind.

Als zwitterionische Tenside werden solche oberflächenaktiven Verbindungen bezeichnet, welche mindestens eine quartäre Ammoniumgruppe und mindestens eine -COO⁽⁻⁾ - oder -SO₃⁽⁻⁾ -Gruppe aufweisen. Besonders geeignete zwitterionische Tenside sind die sogenannten Betaine wie die N-Alkyl-N,N-dimethylammonium-glycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 Kohlenstoffatomen in der Alkyl- oder Acylgruppe sowie das Kokosacylaminoethylhydroxyethylcarboxymethylglycinat. Erfindungsgemäß besonders bevorzugte zwitterionische Tenside sind das unter der INCI-Bezeichnung Cocamidopropyl Betaine bekannte Carbonsäureamid-Derivat sowie Alkylbetaine mit 10 bis 20 Kohlenstoffatomen in der Alkylgruppe.

Gemäß einer bevorzugten Ausführungsform der vorliegenden Erfindung kann das mindestens eine nichtionische Tensid ausgewählt sein aus der Gruppe der polyethoxylierten Carbonsäureester bzw. Fettsäureester mit einer Kettenlänge von 8 bis 30 Kohlenstoffatomen und einem Ethoxylierungsgrad von 5 bis 50, ethoxylierten Glycerylcarbonsäureester mit einem Ethoxylierungsgrad von 2 bis 20, Alkyloligoglucoside mit 8 bis 16 Kohlenstoffatomen in der Alkylgruppe. Weiterhin kann das mindestens eine zwitterionische Tensid ausgewählt sein aus der Gruppe der C₈₋₁₈-Alkylamido(C₁₋₄)-alkylbetaine.

In einer besonders bevorzugten Ausführungsform der vorliegenden Erfindung enthalten die kosmetischen Reinigungszusammensetzungen eine Mischung aus Alkylpolyglykolethersulfaten und Alkylamidoalkylbetainen oder Alkyloligoglucosiden (wie bereits definiert), welche sich durch hervorragende Milde zur Haut sowie exzellente Schaumentwicklung und Schaumqualität auszeichnet.

Als zweite zwingende Komponente b) enthalten die erfindungsgemäßen Reinigungszusammensetzungen mindestens eine teil- und/oder nichtneutralisierte, unverzweigte, gesättigte und/oder ungesättigte Carbonsäure bzw. Fettsäure mit 14 bis 30 Kohlenstoffatomen. Die mindestens eine Carbonsäure kann erfindungsgemäß in einer Menge von 11 bis 22 Gew.-% und insbesondere 12 bis 16 Gew.-%, bezogen auf das Gesamtgewicht der Reinigungszusammensetzung, enthalten sein. Bei Einsatz der zuvor beschriebenen Mengen an teil- und/oder nichtneutralisierten Carbonsäuren b) wird eine optisch und sensorisch ansprechende Textur und ein hoher Pflegecharakter der erfindungsgemäßen Reinigungszusammensetzung erhalten.

Erfindungsgemäß bevorzugte Carbonsäuren b) sind Myristinsäure, Kokossäure, Palmitinsäure, Stearinsäure, Oleinsäure, Elaidinsäure, Petroselinsäure, Linolsäure, Linolensäure, Elaeostearinsäure, Arachinsäure, Gadoleinsäure, Behensäure, Erucasäure, Lignocerinsäure, Cerotinsäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Besonders bevorzugt sind Carbonsäuren mit 16 bis 22 Kohlenstoffatomen. Die vorstehend genannten Carbonsäuren zeigen ein gutes Rückfettungsverhalten, so dass eine Austrocknung der Haut und/oder der Haare selbst bei häufiger Anwendung der Reinigungszusammensetzung vermieden wird.

In diesem Zusammenhang können gemäß einer bevorzugten Ausführungsform der vorliegenden Erfindung Stearinsäure und/oder Palmitinsäure als Carbonsäure b) eingesetzt werden.

Die erfindungsgemäßen Reinigungszusammensetzungen enthalten weiterhin nichtionische Polymere c). Gemäß einer bevorzugten Ausführungsform der vorliegenden Erfindung werden wasserlösliche nichtionische Polymere c) eingesetzt. Im Rahmen der vorliegenden Erfindung sind unter der Bezeichnung "wasserlöslich" solche nichtionischen Polymere c) zu verstehen, welche in 1%-iger Konzentration bei 25°C in Wasser für das menschliche Auge klare oder transluzente Lösungen liefern.

Erfindungsgemäß eingesetzte nichtionische Polymere c) entsprechen der Formel (I), 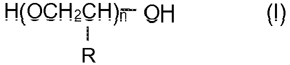 in der R für ein Wasserstoffatom oder eine Methylgruppe, und n für einen ganzzahligen Wert von 1.360 bis 99.000 steht. Besonders bevorzugte nichtionische Polymere c) weisen einen ganzzahligen Wert n von 1.360 bis 97.000, mehr bevorzugt von 1.360 bis 94.000 und insbesondere von 1.360 bis 91.000 auf.

Insbesondere bevorzugt sind Polyethylenglykole mit mittleren Molekulargewichten M_{w} im Bereich von 600.00 bis 10.000.000 Dalton, bevorzugt von 100.000 bis 9.000.000 Dalton, mehr bevorzugt von 300.000 bis 8.000.000 Dalton, noch mehr bevorzugt von 500.000 bis 7.000.000 Dalton und insbesondere von 600.000 bis 4.000.000 Dalton. Das mittlere Molekulargewicht M_{w} kann beispielsweise durch Gelpermeationschromatographie (GPC) mit Polystyrol als internem Standard gemäß DIN 55672-3 bestimmt werden.

Die nichtionischen Polymere c) können in der erfindungsgemäßen Reinigungszusammensetzung in Mengen von 0,1 bis 1 Gew.-%, bezogen auf das Gesamtgewicht der Reinigungszusammensetzung, eingesetzt werden.

Es wurde gefunden, dass diese speziellen nichtionischen Polymere c) das Schaumverhalten der erfindungsgemäßen Reinigungszusammensetzungen signifikant verbessern und selbst bei hohen Gehalten an teil- und/oder nichtneutralisierten Carbonsäuren, welche sich negativ auf das Schaumverhalten auswirken, eine hohe Schaumbildung erzielt wird. Weiterhin unterstützen die hochmolekularen nichtionischen Polymere c) die Rheologie der erfindungsgemäßen Reinigungszusammensetzungen sowie deren Schaumeigenschaften, insbesondere die Feinporigkeit und Cremigkeit des Schaums, so dass eine mousseartige Textur resultiert. Darüber hinaus führt der Einsatz der hochmolekularen nichtionischen Polymere c) zu einer hohen Stabilität der mousseartigen Textur der erfindungsgemäßen Reinigungszusammensetzungen, so dass die Reinigungszusammensetzungen als Mousse verpackt, gelagert und in den Handel gebracht zu werden können. Dadurch kann der Einsatz von umweltschädlichen Treibgasen in Aerosoldosen oder aufwendige Verpackungen wie Pumpdispenser vermieden werden. Auch kann durch den Einsatz der hochmolekularen nichtionischen Polymere c) das unerwünschte glitschige Gefühl des Schaums auf der Haut, welches viele Handelsprodukte aufweisen, vermieden werden.

Gemäß einer bevorzugten Ausführungsform der vorliegenden Erfindung können die Reinigungszusammensetzungen zur Stabilisierung einen anorganischen Verdicker enthalten. Als anorganische Verdickungsmittel können im Rahmen der vorliegenden Erfindung Schichtsilikate (polymere, kristalline Natriumdisilicate) eingesetzt werden. Besonders stabile Schäume mit hervorragenden Schaumeigenschaften werden erhalten, wenn als anorganischer Verdicker Magnesium Aluminium Silikate aus der Gruppe der Bentonite, insbesondere Smektite, wie Montmorillonit oder Hectorit, welche gegebenenfalls auch geeignet modifiziert sein können, eingesetzt werden. Darüber hinaus können auch synthetische Schichtsilikate, wie beispielsweise das von der Firma Süd Chemie unter der Handelsbezeichnung Optigel vertriebene Magnesiumschichtsilikat verwendet werden.

Neben den vorgenannten Bestandteilen können die Reinigungszusammensetzungen eine Reihe weiterer fakultativer Bestandteile enthalten. Bevorzugt werden den erfindungsgemäßen Reinigungszusammensetzungen weitere Wirkstoffe zugesetzt, welche zusätzliche kosmetische Pflegeeigenschaften aufweisen, um die Konditionierung der Haut und/oder der Haare während des Reinigungsvorgangs zu unterstützen. Als solche sind insbesondere kosmetisch geeignete Ölkomponenten, Pflanzenextrakte und/oder Feuchthaltemittel als weitere bevorzugte fakultative Komponenten zu nennen.

Erfindungsgemäß geeignete Ölkomponenten können ausgewählt sein aus mineralischen, natürlichen oder synthetischen Ölkomponenten wie Petrolatum, Paraffinen, Silikonen, Alkoholen, Fettsäureestern, natürlichen Ölen pflanzlichen und tierischen Ursprungs sowie deren Mischungen. Die Ölkomponenten können in einer Menge von 0,005 bis 20 Gew.-%, bevorzugt von 0,01 bis 10 Gew.-%, besonders bevorzugt von 0,05 bis 5 Gew.-% und insbesondere von 0,2 bis 3 Gew.-%, bezogen auf das Gesamtgewicht der Reinigungszusammensetzung und den Gesamtgehalt aller Ölkomponenten, eingesetzt werden.

Unter dem Begriff der Silikone versteht der Fachmann verschiedene Strukturen siliciumorganischer Verbindungen, welche in der erfindungsgemäßen Reinigungszusammensetzung in Mengen von 0,01 bis 3 Gew.-%, bevorzugt von 0,05 bis 2 Gew.-% und insbesondere von 0,1 bis 1 Gew.-%, bezogen auf das Gesamtgewicht der Reinigungszusammensetzung, enthalten sein können.

Insbesondere bevorzugt können die Silikone ausgewählt sein aus mindestens einem Vertreter der Gruppe siliciumorganischer Verbindungen, die gebildet wird aus:
(i) Polyalkylsiloxanen, Polyarylsiloxanen, Polyalkylarylsiloxanen, die flüchtig oder nicht flüchtig, geradkettig, verzweigt oder zyklisch, vernetzt oder nicht vernetzt sind;
(ii) Polysiloxanen, die in ihrer allgemeinen Struktur eine oder mehrere organofunktionelle Gruppen enthalten, die ausgewählt sind aus:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)-Polyoxyalkylen(B)-Blockcopoylmeren vom Typ (A-B)ₙ mit n > 3;
(iv) gepfropften Silikonpolymeren mit nichtsilikonhaltigem, organischem Grundgerüst, die aus einer organischen Hauptkette bestehen, welche aus organischen Monomeren gebildet wird, die kein Silikon enthalten, auf die in der Kette sowie gegebenenfalls an mindestens einem Kettenende mindestens ein Polysiloxanmakromer gepfropft wurde;
(v) gepfropften Silikonpolymeren mit Polysiloxan-Grundgerüst, auf das nichtsilikonhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silikon enthält, wie beispielsweise das unter der INCI-Bezeichnung Bis-PEG/PPG-20/20 Dimethicone vertriebene Handelsprodukt Abil B 8832 der Firma Degussa;
(vi) oder deren Gemischen.

In einer Ausführungsform der vorliegenden Erfindung ist das Konditioniermittel ein konditionierendes Silikon mit einer Viskosität von 20.000 bis 120.000 mPa·s, ganz besonders bevorzugt von 40.000 bis 80.000 mPa·s.

Besonders bevorzugt ist dabei das konditionierende Silikon ausgewählt aus Dimethiconen, Amodimethiconen oder Dimethiconolen.

Als Fettalkohole können gesättigte, ein- oder mehrfach ungesättigte, verzweigte oder unverzweigte Fettalkohole mit C₆-C₃₀-, bevorzugt C₁₀-C₂₂-, und ganz besonders bevorzugt C₁₂-C₂₂-Kohlenstoffatomen eingesetzt werden. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren mit C₆-C₃₀-Kohlenstoffatomen ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol®, z.B. Stenol® 1618 oder Lanette®, z.B. Lanette® O oder Lorol®, z.B. Lorol® C8, Lorol® C14, Lorol® C18, Lorol® C8-18, HD-Ocenol®, Crodacol®, z.B. Crodacol® CS, Novol®, Eutanol® G, Guerbitol® 16, Guerbitol® 18, Guerbitol® 20, Isofol® 12, Isofol® 16, Isofol® 24, Isofol® 36, Isocarb® 12, Isocarb® 16 oder Isocarb® 24 käuflich zu erwerben. Selbstverständlich können erfindungsgemäß auch Wollwachsalkohole, wie sie beispielsweise unter den Bezeichnungen Corona®, White Swan®, Coronet® oder Fluilan® käuflich zu erwerben sind, eingesetzt werden.

Erfindungsgemäß geeignete Fettalkohole werden in der Reinigungszusammensetzung in Mengen von 0,01 bis 3 Gew.-%, bevorzugt in Mengen von 0,05 bis 2 Gew.-% und insbesondere von 0,1 bis 1 Gew.-%, bezogen auf das Gesamtgewicht der Reinigungszusammensetzung, eingesetzt.

Als natürliche oder synthetische Wachse können erfindungsgemäß feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs und Microwachse aus PE- oder PP eingesetzt werden. Derartige Wachse sind beispielsweise erhältlich über die Fa. Kahl & Co., Trittau.

Zu den natürlichen und synthetischen kosmetischen Ölkörpern sind beispielsweise zu zählen:
- flüssige Paraffinöle, Isoparaffinöle und synthetische Kohlenwasserstoffe sowie Di-n-alkylether mit insgesamt zwischen 12 bis 36 Kohlenstoffatomen, insbesondere 12 bis 24 Kohlenstoffatomen, wie beispielsweise Di-n-octylether, Di-n-decylether, Di-n-nonylether, Din-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethylhexyl)-cyclohexan (Cetiol® S) und Di-n-octylether (Cetiol® OE) können bevorzugt sein.
- Esteröle. Unter Esterölen sind zu verstehen die Ester von C₆-C₃₀-Fettsäuren mit C₂-C₃₀-Fettalkoholen. Bevorzugt sind die Monoester der Fettsäuren mit Alkoholen mit 2 bis 24 Kohlenstoffatomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit® IPM), Isononansäure-C₁₆-₁₈-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkoholcaprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).

- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, Glycerincarbonat oder Dicaprylylcarbonat (Cetiol® CC),
- Trifettsäureester von gesättigten und/oder ungesättigten linearen und/oder verzweigten Fettsäuren mit Glycerin,
- Fettsäurepartialglyceride, das sind Monoglyceride, Diglyceride und deren technische Gemische. Bei der Verwendung technischer Produkte können herstellungsbedingt noch geringe Mengen Triglyceride enthalten sein. Die Partialglyceride folgen vorzugsweise der Formel, 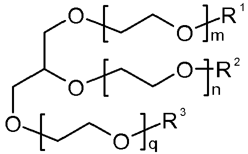 in der R¹, R² und R³ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht R¹ für einen Acylrest und R² und R³ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

In einer besonders bevorzugten Ausführungsform der Erfindung wird als Ölkomponente ein pflanzliches Öl eingesetzt.

Als natürliche Öle kommen beispielsweise Amaranthsamenöl, Aprikosenkernöl, Arganöl, Avocadoöl, Babassuöl, Baumwollsaatöl, Borretschsamenöl, Camelinaöl, Distelöl, Erdnußöl, Granatapfelkernöl, Grapefruitsamenöl, Hanföl, Hagebuttenkernöl, Haselnussöl, Holundersamenöl, Johannisbeersamenöl, Jojobaöl, Kakaobutter, Leinöl, Macadamianussöl, Maiskeimöl, Mandelöl, Marulaöl, Nachtkerzenöl, Olivenöl, Palmöl, Pfirsischkernöl, Rapsöl, Reisöl, Sanddornfruchtfleischöl, Sanddornkernöl, Sesamöl, Sheabutter, Sojaöl, Sonnenblumenöl, Traubenkernöl, Walnußöl oder Wildrosenöl in Frage.

Besonders bevorzugt sind erfindungsgemäß Avocadoöl, Aprikosenkernöl, Hagebuttenkernöl, Jojobaöl, Kakaobutter, Mandelöl, Olivenöl, Pfirsischkernöl, Sheabutter, Sonnenblumenöl und Traubenkernöl.

Die Öle werden in der erfindungemäßen Reinigungszusammensetzung in Mengen von 0,01 bis 3 Gew.-%, bevorzugt von 0,05 bis 2,5 Gew.-% und insbesondere in Mengen von 0,1 bis 2 Gew.-%, bezogen auf das Gesamtgewicht der Reinigungszusammensetzung, eingesetzt.

Unter erfindungsgemäß geeigneten Pflanzenextrakten sind Extrakte zu verstehen, die aus allen Teilen einer Pflanze hergestellt werden können.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Erfindungsgemäß sind vor allem die Extrakte aus Grünem Tee, Weißem Tee, Eichenrinde, Brennnessel, Hamamelis, Hopfen, Kamille, Klettenwurzel, Schachtelhalm, Weißdorn, Lindenblüten, Litschi, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Ginseng, Ingwerwurzel, Echinacea purpurea, Olea europea, Foeniculum vulgaris und Apim graveolens bevorzugt.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 bis 80 Gew.-% Aktivsubstanz und als Lösungsmittel das bei ihrer Gewinnung eingesetzte Extraktionsmittel oder Extraktionsmittelgemisch.

Zusätzlich kann es sich als vorteilhaft erweisen, den erfindungsgemäßen Reinigungszusammensetzungen Feuchthaltemittel bzw. Penetrationshilfsstoffe und/oder Quellmittel zuzusetzen. Diese Hilfsstoffe sorgen für eine bessere Penetration von Wirkstoffen in die keratinische Faser oder helfen die keratinische Faser aufzuquellen. Hierzu zählen beispielsweise Harnstoff und Harnstoffderivate, Guanidin und dessen Derivate, Arginin und dessen Derivate, Wasserglas, Imidazol und dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol. Erfindungsgemäß besonders geeignet ist Glycerin.

Die Feuchthaltemittel werden in der erfindungsgemäßen Reinigungszusammensetzung in Mengen von 0,01 bis 10 Gew.-%, bevorzugt in Mengen von 0,05 bis 5 Gew.-% und insbesondere in Mengen von 0,1 bis 3 Gew.-%, bezogen auf das Gesamtgewicht der Reinigungszusammensetzung, eingesetzt.

Neben den vorgenannten zwingenden erfindungsgemäßen Komponenten und den fakultativen, aber bevorzugten weiteren Komponenten können die erfindungsgemäßen Reinigungszusammensetzungen weitere Stoffe enthalten, welche die Haut und/oder die Haare konditionieren oder die Anwendungseigenschaften der Mittel verbessern.

Zu den weiteren konditionierenden Komponenten sind erfindungsgemäß die kationischen Polymere zu zählen. Unter erfindungsgemäß geeigneten kationischen Polymeren sind Polymere zu verstehen, welche in der Haupt- und/oder Seitenkette "temporär kationische" oder "permanent kationische" Gruppen aufweisen. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten.

Bevorzugte kationische Gruppen enthalten quartäre Ammoniumgruppen. Insbesondere solche Polymere, bei welchen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Besonders bevorzugte kationische Polymere sind Homopolymere der allgemeinen Formel (VI), 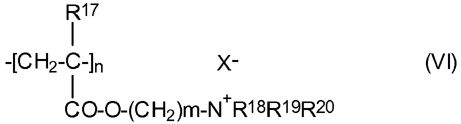 in der R¹⁷ = -H oder -CH₃ ist, R¹⁸, R¹⁹ und R²⁰ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ein physiologisch verträgliches organisches oder anorganisches Anion ist, sowie Copolymere, bestehend im Wesentlichen aus den in Formel (VI) aufgeführten Monomereinheiten sowie nichtionogenen Monomereinheiten.

Im Rahmen der zuvor genannten Polymere sind diejenigen erfindungsgemäß bevorzugt, für welche mindestens eine der folgenden Bedingungen gilt:
- R¹⁷ steht für eine Methylgruppe
- R¹⁸, R¹⁹ und R²⁰ stehen für Methylgruppen
- m hat den Wert 2.

Als physiologisch verträgliche Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Erfindungsgemäß geeignete Homo- oder Copolymere, die sich von der Formel (VI) ableiten, sind beispielsweise die unter den Handelsbezeichnungen Salcare® SC 95, Salcare® SC 96 und Salcare® SC 92 im Handel erhältlichen kationischen Polymere.

Weitere bevorzugte kationische Polymere sind beispielsweise
- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate,
- hydrophob modifizierte Cellulosederivate, beispielsweise die unter dem Handelsnamen SoftCat® vertriebenen kationischen Polymere,
- kationische Alkylpolyglycoside,
- kationiserter Honig, beispielsweise das Handelsprodukt Honeyquat® 50,
- kationische Guar-Derivate, wie insbesondere die unter den Handelsnamen Cos-media®Guar und Jaguar® vertriebenen Produkte,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning® 929 Emulsion (enthaltend ein hydroxylaminomodifiziertes Silikon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat®734 und Gafquat®755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden,
- quaternierter Polyvinylalkohol,
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft® LM 200) bekannten Polymere. Ebenfalls erfindungsgemäß verwendbar sind die Copolymere des Vinylpyrrolidons, wie sie als Handelsprodukte Copolymer 845 (Hersteller: ISP), Gaffix® VC 713 (Hersteller: ISP), Gafquat®ASCP 1011, Gafquat®HS 110, Luviquat®8155 und Luviquat® MS 370 erhältlich sind.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen® CMF, Hydagen® HCMF, Kytamer® PC und Chitolam® NB/101 im Handel frei verfügbar sind. Chitosane sind deacetylierte Chitine, die in unterschiedlichen Deacetylierungsgraden und unterschiedlichen Abbaugraden (Molekulargewichten) im Handel erhältlich sind.

In einer besonders bevorzugten Ausführungsform der Erfindung enthalten die erfindungsgemäßen Reinigungszusammensetzungen zur Steigerung der Hautkonditionierung mindestens ein kationisches Polymer, das ausgewählt wird aus der Gruppe der kationischen Cellulose-Derivate, kationischen Guar-Derivate und/oder Polyquaternium-7 (Merquat 550), Polyquaternium-6, Polyquaternium-10 und/oder Polyquaternium-67 (SoftCat®-Polymere).

Das oder die kationische(n) Polymer(e) ist (sind) in der erfindungsgemäßen Reinigungszusammensetzung in Mengen von 0,1 bis 5 Gew.-%, bezogen auf das Gesamtgewicht der Reinigungszusammensetzung, enthalten. Mengen von 0,2 bis 3 Gew.-%, insbesondere von 0,5 bis 2 Gew.-%, bezogen auf das Gesamtgewicht der Reinigungszusammensetzung, sind besonders bevorzugt.

Die erfindungsgemäßen Reinigungszusammensetzungen eignen sich als kosmetische Zusammensetzungen zur Reinigung der Haut und/oder der Haare und können beispielsweise als Haarshampoo, Duschgel, Duschbad, Waschgel, Gesichtsreiniger, Handwaschmittel und/oder Schaumbad vorliegen. Sie weisen hervorragende Schaumeigenschaften sowie eine mousseartige Textur auf und besitzen aufgrund des hohen Anteils an teil- bzw. nichtneutralisierten Carbonsäuren einen ausgezeichneten Pflegecharakter, so dass selbst bei häufiger Anwendung keine Hauttrockenheit auftritt.

Je nach Anwendungszweck kann die erfindungsgemäße Reinigungszusammensetzung neben den vorgenannten zwingenden und bevorzugten fakultativen Komponenten noch weitere Wirk-, Hilfs- und Zusatzstoffe enthalten, die im folgenden beschrieben werden.

Erfindungsgemäß einsetzbar sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Carbonsäuren mit Triethanolamin, quaternierte Estersalze von Carbonsäuren mit Diethanolalkylaminen und quaternierten Estersalzen von Carbonsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75, Dehyquart®C-4046, Dehyquart® L80 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyldimethylamin dar.

Die kationischen Tenside werden bevorzugt in Mengen von 0,05 bis 10 Gew.-%, bezogen auf das Gesamtgewicht der Reinigungszusammensetzung, eingesetzt. Mengen von 0,1 bis 5 Gew.-%, bezogen auf das Gesamtgewicht der Reinigungszusammensetzung, sind besonders bevorzugt.

In einer weiteren bevorzugten Ausführungsform kann die Wirkung der erfindungsgemäßen Reinigungszusammensetzungen durch Emulgatoren gesteigert werden. Solche Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 Kohlenstoffatomen, an Fettsäuren mit 12 bis 22 Kohlenstoffatomen und an Alkylphenole mit 8 bis 15 Kohlenstoffatomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen, zum Beispiel das im Handel erhältliche Produkt Montanov®68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 Kohlenstoffatomen,
- Sterine. Als Sterine wird eine Gruppe von Steroiden verstanden, welche am Kohlenstoffatom 3 des Steroid-Gerüstes eine Hydroxylgruppe tragen und sowohl aus tierischem Gewebe (Zoosterine) als auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospholipide, die z.B. als Lecithine bzw. Phosphatidylcholine aus z.B. Eidotter oder Pflanzensamen (z.B. Sojabohnen) gewonnen werden, verstanden.
- Fettsäureester von Zuckern und Zuckeralkoholen wie Sorbit,
- Polyglycerine und Polyglycerinderivate wie beispielsweise Polyglycerinpoly-12-hydroxystearat (Handelsprodukt Dehymuls® PGPH),
- Lineare und verzweigte Fettsäuren mit 8 bis 30 Kohlenstoffatomen und deren Na-, K-, Ammonium-, Ca-, Mg- und Zn - Salze,
- Monoester und/oder Gemische aus Monoestern und Diestern des Glycerins mit verzweigten, oder geradkettigen, gesättigten oder ungesättigten Carbonsäuren einer Kohlenstoffkettenlänge von 8 bis 24, bevorzugt von 10 bis 18 und insbesondere von 12 bis 16, die einen Ethoxylierungsgrad von 1 bis 20, bevorzugt von 2 bis 17, besonders bevorzugt von 4 bis 13 und insbesondere von 6 bis 10 aufweisen. Erfindungsgemäß bevorzugt sind die ethoxylierten Glyceryloleate und Glycerylcocoate und insbesondere bevorzugt ist PEG-7 Glyceryl Cocoate, wie es beispielsweise im Handel unter der Bezeichnung Tegosoft® GC oder Cetiol® HE erhältlich ist.

Die Emulgatoren werden bevorzugt in Mengen von 0,1 bis 25 Gew.-%, insbesondere 0,5 bis 15 Gew.-%, bezogen auf das Gesamtgewicht der Reinigungszusammensetzung, eingesetzt.

Prinzipiell können nichtionogene Emulgatoren mit einem HLB-Wert von 8 bis 18 eingesetzt werden. Nichtionogene Emulgatoren mit einem HLB-Wert von 10 bis 15 können erfindungsgemäß bevorzugt sein.

In einer weiteren Ausführungsform der Erfindung können die erfindungsgemäßen Reinigungszusammensetzungen zur weiteren Unterstützung ihrer haut- und haarpflegenden Wirkung zudem Proteinhydrolysate und/oder deren Derivate enthalten. Proteinhydrolysate sind Produktgemische, die durch sauer, basisch oder enzymatisch katalysierten Abbau von Proteinen (Eiweißen) erhalten werden.

Erfindungsgemäß können Proteinhydrolysate pflanzlichen und tierischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan® (Cognis), Promois® (Interorgana), Collapuron® (Cognis), Nutrilan® (Cognis), Gelita-Sol® (Deutsche Gelatine Fabriken Stoess & Co), Lexein® (Inolex) und Kerasol® (Croda) vertrieben.

Erfindungsgemäß bevorzugt sind Proteinhydrolysate pflanzlichen Ursprungs, z. B. Soja-, Mandel-, Reis-, Erbsen-, Kartoffel- und Weizenproteinhydrolysate. Solche Produkte sind beispielsweise unter den Warenzeichen Gluadin® (Cognis), DiaMin® (Diamalt), Lexein® (Inolex) und Crotein® (Croda) erhältlich.

Obwohl der Einsatz der Proteinhydrolysate als solche bevorzugt ist, können an deren Stelle gegebenenfalls auch anderweitig erhaltene Aminosäuregemische oder einzelne Aminosäuren wie beispielsweise Arginin, Lysin, Histidin oder Pyrroglutaminsäure eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon® (Cognis), Gluadin® (Cognis), Lexein® (Inolex), Crolastin® (Croda) oder Crotein® (Croda) vertrieben.

Erfindungsgemäß einsetzbar sind auch kationisierte Proteinhydrolysate, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder von biotechnologisch gewonnenen Proteinhydrolysaten, stammen kann. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder durch eine Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25.000 Dalton, bevorzugt 250 bis 5.000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäßen kationischen Proteinhydrolysate und -derivate seien die unter den INCI-Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed keratin, Hydroxypropyltrimonium Hydrolyzed Rice Bran Protein, Hydroxyproypltrimonium Hydrolyzed Silk, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Silk, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Silk, Quaternium-79 Hydrolyzed Soy Protein, Quaternium-79 Hydrolyzed Wheat Protein. Ganz besonders bevorzugt sind die kationischen Proteinhydrolysate und -derivate auf pflanzlicher Basis.

Die Proteinhydrolysate und deren Derivate werden bevorzugt in Mengen von 0,01 bis 10 Gew.-%, bezogen auf das Gesamtgewicht der Reinigungszusammensetzung, eingesetzt. Mengen von 0,1 bis 5 Gew.-%, insbesondere 0,1 bis 3 Gew.-%, bezogen auf das Gesamtgewicht der Reinigungszusammensetzung, sind ganz besonders bevorzugt.

Ebenfalls als vorteilhaft hat sich die Kombination der erfindungsgemäßen Reinigungszusammensetzung mit Vitaminen, Provitaminen und Vitaminvorstufen sowie deren Derivaten erwiesen.

Dabei sind erfindungsgemäß solche Vitamine, Pro-Vitamine und Vitaminvorstufen bevorzugt, welche üblicherweise den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die Vitamin A-Komponente wird bevorzugt in Mengen von 0,05 bis 1 Gew.-%, bezogen auf das Gesamtgewicht der Reinigungszusammensetzung, eingesetzt.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, welches bevorzugt in Mengen von 0,05 bis 1 Gew.-%, bezogen auf das Gesamtgewicht der Reinigungszusammensetzung, eingesetzt wird.
- Vitamin B₅ (Pantothensäure und Panthenol). Im Rahmen dieser Gruppe wird bevorzugt das Panthenol eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅-Typs werden bevorzugt in Mengen von 0,05 bis 10 Gew.-%, bezogen auf das Gesamtgewicht der Reinigungszusammensetzung, eingesetzt. Mengen von 0,1 bis 5 Gew.-%, bezogen auf das Gesamtgewicht der Reinigungszusammensetzung, sind besonders bevorzugt.
- Vitamin B₆ (Pyridoxin sowie Pyridoxamin und Pyridoxal).

Vitamin C (Ascorbinsäure). Die übliche Einsatzmenge von Vitamin C beträgt 0,1 bis 3 Gew.-%, bezogen auf das Gesamtgewicht der Reinigungszusammensetzung. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, werden erfindungsgemäß bevorzugt in Mengen von 0,05 bis 1 Gew.-%, bezogen auf das Gesamtgewicht der Reinigungszusammensetzung, eingesetzt.

Vitamin F. Unter dem Begriff "Vitamin F" werden üblicherweise essentielle Carbonsäuren, insbesondere Linolsäure, Linolensäure und Arachidonsäure, verstanden.

Vitamin H. Als Vitamin H wird die Verbindung (3aS,4S, 6aR)-2-Oxohexahydrothienol[3,4-d]-imidazol-4-valeriansäure bezeichnet, für die sich zwischenzeitlich der Trivialname Biotin durchgesetzt hat. Biotin wird bevorzugt in Mengen von 0,0001 bis 1,0 Gew.-%, insbesondere in Mengen von 0,001 bis 0,01 Gew.-%, bezogen auf das Gesamtgewicht der Reinigungszusammensetzung, eingesetzt.

Erfindungsgemäß besonders bevorzugt ist der Einsatz von Vitaminen, Provitaminen und Vitaminvorstufen aus den Gruppen A, B, E und H. Panthenol und seine Derivate sowie Nicotinsäureamid und Biotin sind besonders bevorzugt.

Weiterhin kann in einer bevorzugten Ausführungsform der Erfindung zusätzlich ein UV-Filter eingesetzt werden. Die erfindungsgemäß zu verwendenden UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Gemäß einer weiteren Ausführungsform der Erfindung sind solche UV-Filter bevorzugt, welche eine kationische Gruppe, insbesondere eine quartäre Ammoniumgruppe, aufweisen.

Zwei bevorzugte UV-Filter mit kationischen Gruppen sind die als Handelsprodukte erhältlichen Verbindungen Zimtsäureamidopropyl-trimethylammoniumchlorid (Incroquat®UV-283) und Dodecyldimethylaminobenzamidopropyldimethylammoniumtosylat (Escalol® HP 610).

Der oder die UV-Filter werden üblicherweise in Mengen 0,1 bis 5 Gew.-%, bezogen auf das Gesamtgewicht der Reinigungszusammensetzung, eingesetzt. Mengen von 0,4 bis 2,5 Gew.-%, bezogen auf das Gesamtgewicht der Reinigungszusammensetzung, sind bevorzugt.

Hinsichtlich der Art der erfindungsgemäßen kosmetischen Reinigungszusammensetzungen bestehen keine prinzipiellen Einschränkungen. Als Konfektionierung dieser Zusammensetzungen sind beispielsweise Cremes, Lotionen, Lösungen, Wässer, Emulsionen wie W/O-, O/W-, PIT-Emulsionen (Emulsionen nach der Lehre der Phaseninversion, PIT genannt), Mikroemulsionen und multiple Emulsionen, grobe, instabile, ein- oder mehrphasige Schüttelmixturen und Gele geeignet.

Die erfindungsgemäßen Reinigungszusammensetzungen zeichnen sich neben den hervorragend reinigenden, pflegenden und schäumenden Eigenschaften weiterhin durch ihre anwendungs- und herstellungsfreundliche Rheologie und Viskosität aus.

Dazu weisen sie eine Viskosität im Bereich von 5.000 mPas bis 15.000 mPas, bevorzugt von 6.000 mPas bis 12.000 mPas und insbesondere von 7.500 mPas bis 10.500 mPas auf (jeweils gemessen mit einem Haake Viskosimeter Viscotester VT550; 20°C, Messeinrichtung Zylinder MK-2; Schwergeschwindigkeit 8/sek.).

Neben den erfindungsgemäß zwingenden Komponenten und den weiteren, oben genannten bevorzugten Komponenten können prinzipiell alle weiteren, dem Fachmann für solche kosmetischen Zusammensetzungen bekannten Komponenten eingesetzt werden.

Weitere Wirk-, Hilfs- und Zusatzstoffe sind beispielsweise:
- Verdickungsmittel wie Gelatine oder Pflanzengumme, beispielsweise Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, vollsynthetische Hydrokolloide wie z. B. Polyvinylalkohol,
- Strukturanten wie Maleinsäure und Milchsäure,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- Farbstoffe zum Anfärben des Mittels,
- weitere Substanzen zur Einstellung des pH-Wertes, wie beispielsweise α- und β-Hydroxycarbonsäuren,
- Wirkstoffe wie Allantoin und Bisabolol,
- Komplexbildner wie EDTA, NTA, β-Alanindiessigsäure und Phosphonsäuren,
- Ceramide. Unter Ceramiden werden N-Acylsphingosin (Fettsäureamide des Sphingosins) oder synthetische Analogen solcher Lipide (sogenannte Pseudo-Ceramide) verstanden,
- Trübungsmittel wie Latex, Styrol/PVP- und Styrol/Acrylamid-Copolymere,
- Perlglanzmittel wie Ethylenglykolmono- und -distearat sowie PEG-3-distearat,
- Pigmente,
- Treibmittel wie Propan-Butan-Gemische, N₂O, Dimethylether, CO₂ und Luft,
- Viskositätsregler wie Salze (NaCl).

Der pH-Wert der erfindungsgemäßen Reinigungszusammensetzungen liegt idealerweise in einem hautschonenden Bereich von etwa 4 bis 6, insbesondere in einem Bereich von 4,5 bis 5,5.

Gemäß einer weiteren Ausführungsform der vorliegenden Erfindung kann die erfindungsgemäße Reinigungszusammensetzung weiterhin ein Gas enthalten. Das Gas kann hierbei ausgewählt sein aus der Gruppe von Luft, Argon, Stickstoff, Propan, Butan, N₂O, Dimethylether, CO₂ sowie deren Mischungen. Erfindungsgemäß bevorzugt wird als Gas Luft oder ein Gemisch aus Luft und Treibgasen, wie Propan und/oder Butan, verwendet.

In diesem Zusammenhang kann die Menge des Gases in der Reinigungszusammensetzung von 5 bis 80 Gew.-%, bevorzugt von 5 bis 60 Gew.-%, mehr bevorzugt von 5 bis 40 Gew.-% und insbesondere von 5 bis 20 Gew.-%, bezogen auf das Gesamtgewicht der Reinigungszusammensetzung, betragen. Die Menge des in der Reinigungszusammensetzung eingeschlossenen Gases ergibt sich aus der Gewichtsdifferenz eines identischen Volumens einer Reinigungszusammensetzung mit und ohne eingeschlossenem Gas.

Ein zweiter Gegenstand der Erfindung ist die Verwendung der erfindungsgemäßen - zuvor beschriebenen - Reinigungszusammensetzungen zur Reinigung und Pflege von Haut und Haaren. Bezüglich der Verwendung der erfindungsgemäßen Reinigungszusammensetzung gilt mutatis mutandis das zu den erfindungsgemäßen Reinigungszusammensetzungen gesagte.

Die folgenden Beispiele erläutern die Erfindung, ohne sie jedoch darauf einzuschränken:

### Beispiele

Es wurden die folgende Reinigungszusammensetzungen hergestellt, wobei alle Zahlenwerte in den nachfolgenden Tabellen 1 und 2 - sofern nicht anders angegeben - der Menge des jeweiligen Rohstoffs in Gewichtsprozent entsprechen:

**Tabelle 1: erfindungsgemäße Reinigungszusammensetzungen**

| | **Rezepturen** | | | | | | | | |
|---|---|---|---|---|---|---|---|---|---|
| **Rohstoff** | **I** | **II** | **III** | **IV** | **V** | **VI** | **VII** | **VIII** | **IX** |
| **Fettalkoholpolyglykolethersulfat (C12-14), 2EO, 70% AS** | 12 | 12 | 12 | | 12 | 12 | 12 | 12 | |
| **Natriumlaurylsulfat AS** | | | | 10 | | | | | |
| **Dinatriumlaurethsulfosuccinat 40% AS** | | | | | | | | | 30 |
| **Kokosamidopropylbetain 40% AS** | 8 | 8 | | 8 | 8 | 8 | 8 | 8 | 8 |
| **Plantacare® 818UP** | | | 6 | | | | | | |
| **Polyox**® **WSR-301** | 0,1 | | | | | | 0,1 | | |
| **Polyox**® **WSR N-12K** | | 0,1 | 0,1 | 0,1 | | | | | |
| **Polyox**® **WSR N-60K** | | | | | 0,1 | | | | |
| **Polyox**® **WSR-205** | | | | | | 0,1 | | 0,1 | 0,1 |
| **Cutina**® **FS 45** | 15 | 15 | 15 | | 15 | 15 | 15 | 15 | 15 |
| **Stearinsäure** | | | | 15 | | | | | |
| **Isopropylpalmitat** | 0,5 | 0,5 | 0,5 | | 0,5 | | | | |
| **Isopropylmyristat** | | | | | | 0,5 | | 0,5 | 0,5 |
| **PEG-40 Stearat** | 0,5 | 0,5 | 0,5 | | 0,5 | 0,5 | | | |
| **Cetiol**® **HE** | | | | | | | | 0,5 | 0,5 |
| **Natriumbenzoat** | 0,4 | 0,4 | 0,4 | 0,4 | 0,4 | 0,4 | 0,4 | 0,4 | 0,4 |
| **Citronensäure** | 0,25 | 0,25 | 0,25 | 0,25 | 0,25 | 0,25 | 0,25 | 0,25 | 0,25 |
| **Natriumhydroxid 50%** | 0,117 | 0,117 | 0,117 | 0,117 | 0,117 | 0,117 | 0,117 | 0,117 | 0,117 |
| **Wasser** | ad 100 | ad 100 | ad 100 | ad 100 | ad 100 | ad 100 | ad 100 | ad 100 | ad 100 |

**Tabelle 2: nichterfindungsgemäße Reinigungszusammensetzungen**

| | **Rezepturen** | |
|---|---|---|
| **Rohstoff** | **X** | **XI** |
| **Fettalkoholpolyglykolethersulfat (C12-14), 2EO, 70% AS** | 12 | 12 |
| **Kokosamidopropylbetain 40% AS** | 8 | 8 |
| **Cocomonoethanolamid** | 2 | |
| **Polymer JR 400®** | | 2 |
| **Cutina® FS 45** | 15 | 15 |
| **Isopropylpalmitat** | 0,5 | 0,5 |
| **PEG-40 Stearat** | 0,5 | 0,5 |
| **Natriumbenzoat** | 0,4 | 0,4 |
| **Citronensäure** | 0,25 | 0,25 |
| **Natriumhydroxid 50%** | 0,117 | 0,117 |
| **Wasser** | ad 100 | ad 100 |

Es wurden die folgenden Handelsprodukte eingesetzt:

| | |
|---|---|
| Cocomonoethanolamid: | INCI-Bezeichnung: Cocamide MEA; Cognis |
| Cetiol® HE: | INCI-Bezeichnung: PEG-7 Glyceryl Cocoate; Cognis |
| Plantacare® 818UP: | INCI-Bezeichnung: Coco-Glucoside, ca. 50 % Aktivsubstanz; Cognis |
| Polymer JR 400®: | INCI-Bezeichnung: Polyquaternium-10, UNION CARBIDE |
| Cutina® FS 45: | INCI-Bezeichnung: Stearic Acid (and) Palmitic Acid, Cognis |
| Polyox® WSR-301: | INCI-Bezeichnung: PEG-90M, Dow Chemical Company |
| Polyox® WSR N-12K | INCI-Bezeichnung: PEG-23M, Dow Chemical Company |
| Polyox® WSR N-60K | INCI-Bezeichnung: PEG-45M, Dow Chemical Company |
| Polyox® WSR-205 | INCI-Bezeichnung: PEG-14M, Dow Chemical Company |

Die Herstellung der Reinigungszusammensetzungen I bis XI erfolgt nach folgender allgemeiner Vorschrift:
Die Waschrohstoffe sowie die Hauptmenge des Wassers werden vorgelegt, auf 70 °C bis 80 °C erwärmt und bis zur Homogenität verrührt. Anschließend werden gegebenenfalls die in warmem Wasser vorgequollenen Verdicker sowie polymeren Pflegestoffe hinzugefügt. Nachdem alle Inhaltsstoffe hinzugefügt sind, kann gegebenenfalls eine Begasung mit Gas unter Kühlung und Rühren auf 25 °C erfolgen. Ist keine Begasung vorgesehen, wird die Mischung unter Rühren auf 25 °C abgekühlt. Die Konservierungsmittel sowie gegebenenfalls mit einem Lösungsvermittler vermischte Parfümöle werden erst bei einer Temperatur von 35 °C zugegebenen. Nach Abkühlung der Reinigungszusammensetzung erfolgt die Einstellung des pH-Wertes mit Citronensäure.

### Ergebnisse:

Die erfindungsgemäßen Reinigungszusammensetzungen I bis IX (vgl. Tabelle 1) resultieren in einer optisch ansprechenden mousseartigen Textur und weisen hervorragende Schaumeigenschaften in Bezug auf die Aufschäumbarkeit, Verteilbarkeit, Schaummenge, Porengröße, Cremigkeit, Festigkeit, Stabilität und das Abspülvermögen auf.

Der Einsatz von im Stand der Technik zur Schaumsteigerung verwendeten kationischen Polymeren (nichterfindungsgemäße Reinigungszusammensetzungen X und XI, vgl. Tabelle 2) resultiert in einer signifikant schlechteren Schaumbildung, so dass keine mousseartige Textur erhalten wird. Weiterhin weist der Schaum eine äußerst geringe Stabilität auf.

Die Schaumeigenschaften der erfindungsgemäßen Reinigungszusammensetzungen I bis IX wurden von einem Team von Experten in einem Blindtest gegenüber den nichterfindungsgemäßen Reinigungszusammensetzungen, welche auf kationischen Polymeren basieren, als verbessert wahrgenommen.

Die Reinigung der Haut mit den erfindungsgemäßen Reinigungszusammensetzungen I bis IX führte zu effektiv gereinigter Haut, welche sich aufgrund des hohen Anteils and nicht- bzw. teilneutralisierten Fettsäuren weich und gepflegt anfühlte.

## Patentansprüche

1. Kosmetische Reinigungszusammensetzung, enthaltend
a) 0,1 bis 30 Gew.-%, bezogen auf das Gesamtgewicht der Reinigungszusammensetzung, Tensid, ausgewählt aus der Gruppe der anionischen, nichtionischen und/oder zwitterionischen Tenside sowie deren Mischungen,
b) mindestens eine nichtneutralisierte, unverzweigte, gesättigte und/oder ungesättigte Carbonsäure mit einer Kettenlänge von 14 bis 30 Kohlenstoffatomen in einer Menge von 11 bis 22 Gew.-%, bezogen auf das Gesamtgewicht der Reinigungszusammensetzung und
c) mindestens ein nichtionisches Polymer der Formel (I) in einer Menge von 0,1 bis 1 Gew.-%, bezogen auf das Gesamtgewicht der Reinigungszusammensetzung, 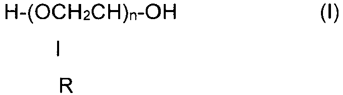 wobei R für ein Wasserstoffatom oder eine Methylgruppe und n für einen ganzzahligen Wert von 1.360 bis 99.000 steht.

2. Reinigungszusammensetzung nach Anspruch 1, **dadurch gekennzeichnet, dass** mindestens ein anionisches und mindestens ein nichtionisches und/oder zwitterionisches Tensid enthalten ist, wobei das Gewichtsverhältnis des(r) anionischen Tensids(e) zu dem(n) nichtionischen und/oder zwitterionischen Tensid(en) 3 : 1 bis 1 : 2, bevorzugt 2 : 1 bis 1 : 1,5 und insbesondere 1,5 : 1 bis 1 : 1 beträgt.

3. Reinigungszusammensetzung nach einem der Ansprüche 1 oder 2, **dadurch gekennzeichnet, dass** das anionische Tensid in einer Menge von 0,3 bis 30 Gew.-%, bevorzugt 0,4 bis 20 Gew.-% und insbesondere 0,5 bis 15 Gew.-%, bezogen auf das Gesamtgewicht der Reinigungszusammensetzung, enthalten ist.

4. Reinigungszusammensetzung nach einem der vorangehenden Ansprüche, **dadurch gekennzeichnet, dass** nichtionische und/oder zwitterionische Tensid in einer Menge von 0,1 bis 20 Gew.-%, bevorzugt 0,2 bis 18 Gew.-%, mehr bevorzugt 0,3 bis 15 Gew.-% und insbesondere 0,5 bis 10 Gew.-%, bezogen auf das Gesamtgewicht der Reinigungszusammensetzung, enthalten ist.

5. Reinigungszusammensetzung nach einem der vorangehenden Ansprüche, **dadurch gekennzeichnet, dass** das mindestens eine anionische Tensid ausgewählt ist aus der Gruppe der Alkylpolyglykolethersulfate, Alkylsulfate und/oder Sulfobernsteinsäuremonoalkylpolyoxyethylester mit 8 bis 18 Kohlenstoffatomen in der Alkylgruppe und 1 bis 10, bevorzugt 1 bis 4 Oxyethylgruppen

6. Reinigungszusammensetzung nach einem der vorangehenden Ansprüche, **dadurch gekennzeichnet, dass** das mindestens eine nichtionische Tensid ausgewählt ist aus der Gruppe der polyethoxylierten Carbonsäureester mit einer Kettenlänge von 8 bis 30 Kohlenstoffatomen und einem Ethoxylierungsgrad von 5 bis 50, ethoxylierten Glycerylcarbonsäureester mit einem Ethoxylierungsgrad von 2 bis 20, Alkyloligoglucoside mit 8 bis 16 Kohlenstoffatomen in der Alkylgruppe

7. Reinigungszusammensetzung nach einem der vorangehenden Ansprüche, **dadurch gekennzeichnet, dass** das mindestens eine zwitterionische Tensid ausgewählt ist aus der Gruppe der C₈₋₁₈-Alkylamido(C₁₋₄)-alkylbetaine.

8. Reinigungszusammensetzung nach einem der vorangehenden Ansprüche, **dadurch gekennzeichnet, dass** die mindestens eine Carbonsäure b) in einer Menge von 12 bis 16 Gew.-%, bezogen auf das Gesamtgewicht der Reinigungszusammensetzung, enthalten ist.

9. Reinigungszusammensetzung nach einem der vorangehenden Ansprüche, **dadurch gekennzeichnet, dass** die mindestens eine Carbonsäure b) ausgewählt ist aus der Gruppe der Myristinsäure, Kokossäure, Palmitinsäure, Stearinsäure, Oleinsäure, Elaidinsäure, Petroselinsäure, Linolsäure, Linolensäure, Elaeostearinsäure, Arachinsäure, Gadoleinsäure, Behensäure, Erucasäure, Lignocerinsäure, Cerotinsäure sowie deren Mischungen

10. Reinigungszusammensetzung nach einem der vorangehenden Ansprüche, **dadurch gekennzeichnet, dass** als Carbonsäure b) Stearinsäure und/oder Palmitinsäure enthalten ist.

11. Reinigungszusammensetzung nach einem der vorangehenden Ansprüche, **dadurch gekennzeichnet, dass** das nichtionische Polymer c) ausgewählt ist aus der Gruppe der Polyethylenglykole, wobei n in der Formel (I) für einen ganzzahligen Wert von 1.360 bis 99.000, bevorzugt von 1.360 bis 97.000, mehr bevorzugt von 1.360 bis 94.000 und insbesondere von 1.360 bis 91.000 steht.

12. Reinigungszusammensetzung nach einem der vorangehenden Ansprüche, **dadurch gekennzeichnet, dass** die Reinigungszusammensetzung weiterhin mindestens einen haar- und/oder hautkonditionierenden Wirkstoff enthält, wobei der Wirkstoff ausgewählt ist aus der Gruppe der Ölkomponenten, kationischen Polymere, Pflanzenextrakte und/oder Feuchthaltemittel.

13. Reinigungszusammensetzung nach einem der vorangehenden Ansprüche, **dadurch gekennzeichnet, dass** die Reinigungszusammensetzung einen pH-Wert im Bereich von 4 bis 6, bevorzugt von 4,5 bis 5,5 aufweist.

14. Verwendung einer Reinigungszusammensetzung nach einem der Ansprüche 1 bis 13 zur Reinigung und Pflege von Haut und Haaren.

## Claims

1. A cosmetic cleansing composition, containing
a) from 0.1 to 30 wt.%, based on the total weight of the cleansing composition, of a surfactant selected from the group consisting of anionic, non-ionic and/or zwitterionic surfactants, and mixtures thereof,
b) at least one non-neutralized, unbranched, saturated and/or unsaturated carboxylic acid having a chain length of from 14 to 30 carbon atoms in an amount of from 11 to 22 wt.%, based on the total weight of the cleansing composition, and
c) at least one non-ionic polymer of formula (I) in an amount of from 0.1 to 1 wt.%, based on the total weight of the cleansing composition, 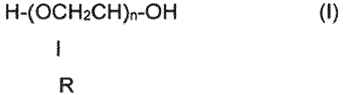 where R represents a hydrogen atom or a methyl group, and n represents a whole number value of from 1,360 to 99,000.

2. The cleansing composition according to claim 1, **characterized in that** at least one anionic and at least one non-ionic and/or zwitterionic surfactant is contained, the weight ratio of the anionic surfactant(s) to the non-ionic and/or zwitterionic surfactant(s) being from 3:1 to 1:2, preferably from 2:1 to 1:1.5, and in particular from 1.5:1 to 1:1.

3. The cleansing composition according to one of claims 1 or 2, **characterized in that** the anionic surfactant is contained in an amount of from 0.3 to 30 wt.%, preferably from 0.4 to 20 wt.%, and in particular from 0.5 to 15 wt.%, based on the total weight of the cleansing composition.

4. The cleansing composition according to one of the preceding claims, **characterized in that** the non-ionic and/or zwitterionic surfactant is contained in an amount of from 0.1 to 20 wt.%, preferably from 0.2 to 18 wt.%, more preferably from 0.3 to 15 wt.%, and in particular from 0.5 to 10 wt.%, based on the total weight of the cleansing composition.

5. The cleansing composition according to one of the preceding claims, **characterized in that** the at least one anionic surfactant is selected from the group consisting of alkyl polyglycol ether sulfates, alkyl sulfates and/or sulfosuccinic acid monoalkyl polyoxyethyl esters having from 8 to 18 carbon atoms in the alkyl group and having from 1 to 10, preferably from 1 to 4 oxyethyl groups.

6. The cleansing composition according to one of the preceding claims, **characterized in that** the at least one non-ionic surfactant is selected from the group consisting of polyethoxylated carboxylic acid esters having a chain length of from 8 to 30 carbon atoms and a degree of ethoxylation of from 5 to 50, ethoxylated glyceryl carboxylic acid esters having a degree of ethoxylation of from 2 to 20, and alkyl oligoglucosides having from 8 to 16 carbon atoms in the alkyl group.

7. The cleansing composition according to one of the preceding claims, **characterized in that** the at least one zwitterionic surfactant is selected from the group consisting of C₈₋₁₈ alkylamido (C₁₋₄) alkylbetaines.

8. The cleansing composition according to one of the preceding claims, **characterized in that** the at least one carboxylic acid b) is contained in an amount of from 12 to 16 wt.%, based on the total weight of the cleansing composition.

9. The cleansing composition according to one of the preceding claims, **characterized in that** the at least one carboxylic acid b) is selected from the group consisting of myristic acid, coconut acid, palmitic acid, stearic acid, oleic acid, elaidic acid, petroselic acid, linoleic acid, linolenic acid, eleostearic acid, arachidic acid, gadoleic acid, behenic acid, erucic acid, lignoceric acid, cerotic acid, and mixtures thereof.

10. The cleansing composition according to one of the preceding claims, **characterized in that** stearic acid and/or palmitic acid is contained as the carboxylic acid b).

11. The cleansing composition according to one of the preceding claims, **characterized in that** the non-ionic polymer c) is selected from the group consisting of polyethylene glycols, where n in the formula (I) represents a whole number value from 1,360 to 99,000, preferably from 1,360 to 97,000, more preferably from 1,360 to 94,000, and in particular from 1,360 to 91,000.

12. The cleansing composition according to one of the preceding claims, **characterized in that** the cleansing composition also contains at least one hair-conditioning and/or skin-conditioning active ingredient, the active ingredient being selected from the group consisting of oil components, cationic polymers, plant extracts and/or humectants.

13. The cleansing composition according to one of the preceding claims, **characterized in that** the cleansing composition has a pH value in the range of from 4 to 6, preferably from 4.5 to 5.5.

14. The use of a cleansing composition according to one of claims 1 to 13 for cleansing and nourishing skin and hair.

## Revendications

1. Composition cosmétique de nettoyage, contenant
a) 0,1 à 30 % en poids, par rapport au poids total de la composition cosmétique, de tensioactif, sélectionné dans le groupe des tensioactifs anioniques, non ioniques et/ou zwitterioniques, ainsi que des mélanges de ceux-ci,
b) au moins un acide carboxylique non neutralisé, non ramifié, saturé et/ou insaturé ayant une longueur de chaîne de 14 à 30 atomes de carbone dans une quantité allant de 11 à 22 % en poids, par rapport au poids total de la composition de nettoyage et
c) au moins un polymère non ionique de formule (I) dans une quantité allant de 0,1 à 1 % en poids, par rapport au poids total de la composition de nettoyage, 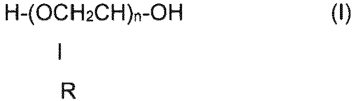 où R représente un atome d'hydrogène ou un groupe méthyle et n représente un nombre entier allant de 1 360 à 99 000.

2. Composition de nettoyage selon la revendication 1, **caractérisée en ce qu'**au moins un tensioactif anionique et au moins un tensioactif non ionique et/ou zwitterionique sont présents, le rapport pondéral entre le(s) tensioactif(s) anionique(s) et le(s) tensioactif(s) non ionique(s) et/ou zwitterionique(s) étant de 3 :1 à 1 :2, de préférence 2 :1 à 1 :1,5 et en particulier 1,5 :1 à 1 :1.

3. Composition de nettoyage selon l'une quelconque des revendications 1 ou 2, **caractérisée en ce que** le tensioactif anionique est présent dans une quantité de 0,3 à 30 % en poids, de préférence de 0,4 à 20 % en poids et en particulier de 0,5 à 15 % en poids, par rapport au poids total de la composition de nettoyage.

4. Composition de nettoyage selon l'une quelconque des revendications précédentes, **caractérisée en ce que** le tensioactif non ionique et/ou zwitterionique est présent dans une quantité de 0,1 à 20 % en poids, de préférence de 0,2 à 18 % en poids, de manière davantage préférée de 0,3 à 15 % en poids et en particulier de 0,5 à 10 % en poids, par rapport au poids total de la composition de nettoyage.

5. Composition de nettoyage selon l'une quelconque des revendications précédentes, **caractérisée en ce que** l'au moins un tensioactif anionique est sélectionné dans le groupe des alkylpolyglycoléthersulfates, alkylsulfates et/ou monoalkylpolyoxyéthylesters de l'acide sulfosuccinique comportant 8 à 18 atomes de carbone dans le groupe alkyle et 1 à 10, de préférence 1 à 4 groupes oxyéthyle.

6. Composition de nettoyage selon l'une quelconque des revendications précédentes, **caractérisée en ce que** l'au moins un tensioactif non ionique est sélectionné dans le groupe des esters de l'acide carboxylique polyéthoxylés ayant une longueur de chaîne de 8 à 30 atomes de carbone et un degré d'éthoxylation de 5 à 50, des esters de l'acide carboxylique et du glycéryle éthoxylés, à un degré d'éthoxylation de 2 à 20, des alkyloligoglucosides comportant 8 à 16 atomes de carbone dans le groupe alkyle.

7. Composition de nettoyage selon l'une quelconque des revendications précédentes, **caractérisée en ce que** l'au moins un tensioactif zwitterionique est sélectionné dans le groupe des C₈₋₁₈-alkylamido(C₁₋₄)-alkylbétaïnes.

8. Composition de nettoyage selon l'une quelconque des revendications précédentes, **caractérisée en ce que** l'au moins un acide carboxylique b) est présent dans une quantité de 12 à 16 % en poids, par rapport au poids total de la composition de nettoyage.

9. Composition de nettoyage selon l'une quelconque des revendications précédentes, **caractérisée en ce que** l'au moins un acide carboxylique b) est sélectionné dans le groupe composé de l'acide myristique, l'acide de noix de coco, l'acide palmitique, l'acide stéarique, l'acide oléique, l'acide élaïdique, l'acide pétrosélinique, l'acide linoléique, l'acide linolénique, l'acide éléostéarique, l'acide icosanoïque, l'acide gadoléique, l'acide béhénique, l'acide érucique, l'acide lignocérique, l'acide cérotique, et des mélanges de ceux-ci.

10. Composition de nettoyage selon l'une quelconque des revendications précédentes, **caractérisée en ce que** l'acide carboxylique b) présent est de l'acide stéarique et/ou de l'acide palmitique.

11. Composition de nettoyage selon l'une quelconque des revendications précédentes, **caractérisée en ce que** le polymère non ionique c) est sélectionné dans le groupe des polyéthylène glycols, n représentant dans la formule (I) un nombre entier allant de 1 360 à 99 000, de préférence de 1 360 à 97 000, de manière davantage préférée de 1 360 à 94 000, et en particulier de 1 360 à 91 000.

12. Composition de nettoyage selon l'une quelconque des revendications précédentes, **caractérisée en ce que** la composition de nettoyage contient en outre au moins un principe actif de conditionnement des cheveux et/ou de la peau, le principe actif étant sélectionné dans le groupe des composants huileux, polymères cationiques, extraits de plante et/ou agents humectants.

13. Composition de nettoyage selon l'une quelconque des revendications précédentes, **caractérisée en ce que** la composition de nettoyage présente une valeur de pH comprise dans une plage allant de 4 à 6, de préférence de 4,5 à 5,5.

14. Utilisation d'une composition de nettoyage selon l'une quelconque des revendications 1 à 13 pour le nettoyage et le soin de la peau et des cheveux.
